# EUROPEAN PATENT APPLICATION

(11) **EP 3 192 502 A1**
(43) Date of publication of application: **19.07.2017**
(21) Application number: 16151562.2
(22) Date of filing: 15.01.2016
(51) Int. Cl.: A61K 9/16

(54) **PHARMACEUTICAL COMPOSITION OF SELEXIPAG**

(71) Applicant: SANDOZ AG, 4056 Basel (CH)
(72) Inventor: The designation of the inventor has not yet been filed
(74) Representative: Kluschanzoff, Harald

(57) **Abstract**

The present invention relates to an amorphous solid dispersion comprising selexipag and methods for preparing the same. The invention also concerns a pharmaceutical composition comprising an effective amount of said amorphous solid dispersion and at least one further pharmaceutically acceptable. The pharmaceutical composition of the present invention may be used as a medicine in particular for the treatment of pulmonary arterial hypertension.

## Description

### FIELD OF THE INVENTION

The present invention relates to a solid dispersion comprising amorphous selexipag and methods for preparing the same. The invention also concerns a pharmaceutical composition comprising an effective amount of said solid dispersion and at least one further pharmaceutically acceptable excipient as well as methods for its preparation. The pharmaceutical composition of the present invention may be used as a medicine in particular for the treatment of pulmonary arterial hypertension.

### BACKGROUND OF THE INVENTION

The diphenylpyrazin derivative selexipag known under the chemical name 2-[4-[*N*-(5,6-diphenylpyrazin-2-yl)-*N-*isopropylamino]butyloxy]-*N-*(methylsulfonyl)acetamide can be represented by the following chemical structure according to Formula (A):

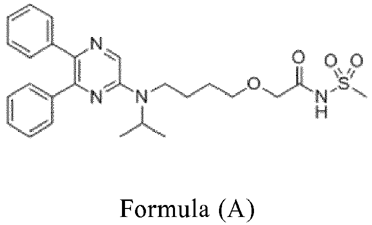

Selexipag, previously also known as NS-304 or ACT-293987, is a potent orally available and highly selective long acting non-prostanoid prostaglandin I2 (PGI-2) receptor agonist, and has been filed for approval in the E.U. and in the U.S. by Actelion for the treatment of pulmonary arterial hypertension. As a prodrug it is *in vivo* transformed by the liver to its active metabolite.

Phase II clinical trials are also ongoing for the treatment of chronic thromboembolic pulmonary hypertension and arteriosclerosis obliterans. Selexipag stimulates PGI-2 receptors in blood vessels and has been described to show, for example, vasodilating effects, bronchodilative effects and a lipid deposition inhibitory effect.

Selexipag and its synthesis are described in WO 2002/088084 A1. In example 84 selexipag is isolated after chromatographic purification. Its physical form in this example is not described.

WO 2010/150865 A1 discloses three crystalline forms of selexipag, designated form I, form II and form III. It is described that these forms were obtained either in pure form or as binary mixtures from a polymorph screening using 32 different solvents and 16 different solvent mixtures (see test example 4). The crystalline forms of WO 2010/150865 are described as useful for the preparation of pharmaceutical formulations and oral dosage forms, such as tablets comprising crystalline selexipag, are described. It should be noted that amorphous selexipag was only described in one single experiment using 1,3,5-trimethylbenzene as solvent (table 4, experiment 30) in WO 2010/150865.

In general, amorphous forms are thermodynamically metastable with respect to the crystalline state. Above their glass transition temperatures (Tg) amorphous materials show a high tendency towards crystallization. Crystallization is often accelerated by water adsorption because the plasticizing effect of water results in a decrease of the glass transition temperature.

While amorphous solids have increased solubility and an accelerated dissolution rate in comparison to their crystalline counterparts, crystalline solid forms of an active pharmaceutical ingredient are often preferred for the preparation of pharmaceutical compositions, such as tablets, for various reasons such as stability with respect to the solid form or chemical stability or ease of handling during formulation of a pharmaceutical. As a consequence, slower dissolution and lower bioavailability of the drug are consciously accepted and pharmaceutical compositions, which are maybe not optimal with regard to these parameters, are produced and marketed.

### SUMMARY OF THE INVENTION

The present inventors have realized that selexipag has a high tendency to crystallize. The present inventors have then managed to prepare stable pharmaceutical compositions comprising amorphous selexipag despite selexipag's high tendency to crystallize. The pharmaceutical compositions of the present invention are advantageous compared to pharmaceutical compositions comprising crystalline selexipag in that their faster dissolution can lead to a faster onset of action, which can be beneficial for the treatment of a disease like pulmonary arterial hypertension.

One objective of the present invention is thus to provide a pharmaceutical composition comprising an effective amount of amorphous selexipag which retains its amorphous character upon storage, for example throughout its shelf-life.

It was found that selexipag has a high tendency to crystallize. It was then surprisingly found that amorphous selexipag can be stabilized in the context of a solid dispersion further comprising at least one pharmaceutically acceptable excipient, wherein the at least one pharmaceutically acceptable excipient is (i) a polymer, or (ii) a silicon-based inorganic adsorbent. For example, a solid dispersion with a particular polymer or using silica carriers and amorphous selexipag can be formed in that way. Therefore, the present invention relates to solid dispersions of amorphous selexipag with specific polymer and silica carriers respectively and to methods for their preparation. The invention also relates to a pharmaceutical composition comprising an effective amount of amorphous selexipag, and to a method for the preparation thereof.

### Definitions

As used herein the term "room temperature" refers to temperatures in the range of from 20 to 30 °C.

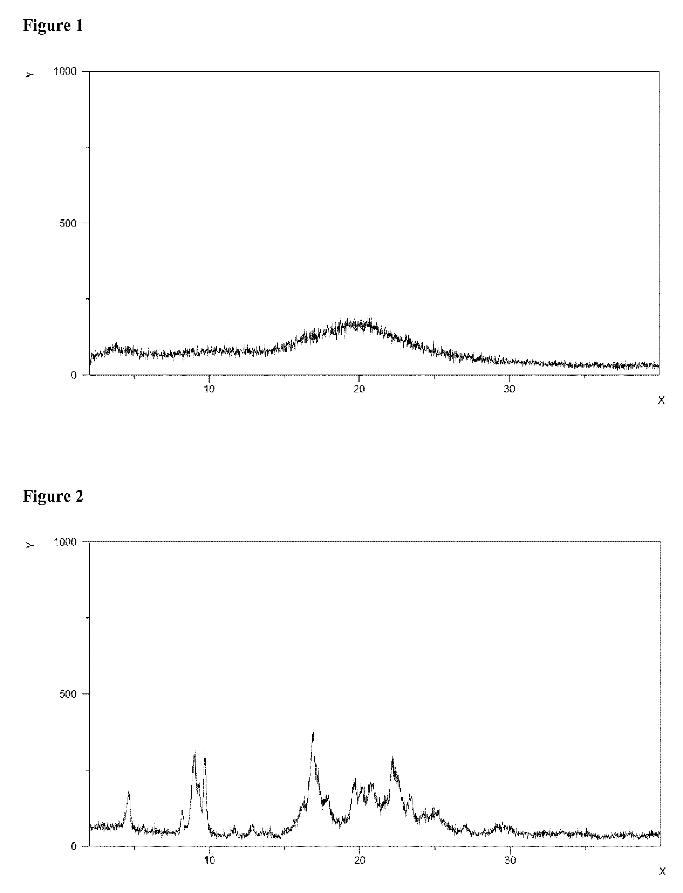

As used herein, the term "amorphous" is used for non-crystalline material, which lacks long-range inter-molecular order. Amorphous solids are generally isotopic, i.e. exhibit similar properties in all directions. When analyzed by powder X-ray diffraction, no sharp characteristic peaks are observed, but instead one or several broad peaks (e.g. "halos") appear. Figure 1 is an example of a powder X-ray diffraction pattern of an amorphous solid.

The term "solid dispersion" as used herein refers to a system in a solid state (as opposed to a liquid or gaseous state) wherein one component is dispersed more or less evenly throughout the other component or components, e.g. in the context of the present invention amorphous selexipag within the pharmaceutically acceptable excipient, wherein the at least one pharmaceutically acceptable excipient is (i) a polymer, or (ii) a silicon-based inorganic adsorbent. Preferred solid dispersions are "solid solutions", where the dispersion of the components is such that the system is chemically and physically uniform or homogeneous throughout or even consists of one phase as defined by measurement of thermodynamic properties of the system, e.g. the amorphous selexipag and the pharmaceutically acceptable excipient, wherein the at least one pharmaceutically acceptable excipient is (i) a polymer, or (ii) a silicon-based inorganic adsorbent, form a system that is chemically and physically uniform or homogeneous throughout or even consists of one phase as defined by measurement of thermodynamic properties of the system.

As used herein the term "glass transition temperature" corresponds to a change in physical state from a glassy (rigid, highly viscous, brittle) material to a more fluid or malleable rubbery state ("Polymorphism in the Pharmaceutical Industry", Chapter 10.4.1 "The amorphous state" Petit S., Coquerel G., Wiley edited by Hilfiker R.).

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order (also see explanation above), thus resulting in broad scattering. According to literature, long-range order e.g. extends over approximately more than 100 atoms, whereas short-range order is over a few atoms only (see "Fundamentals of Powder Diffraction and Structural Characterization of Materials by Vitalij K. Pecharsky and Peter Y. Zavalij, Kluwer Academic Publishers, 2003, page 3).

The term "essentially the same" with reference to powder X-ray diffraction (PXRD) means that variabilities in peak positions and relative intensities of the peaks are to be taken into account. For example, a typical precision of the 2-Theta values is in the range of ± 0.2° 2-Theta. Thus, a diffraction peak that usually appears at 9.0° 2-Theta for example can appear between 8.8° and 9.2° 2-Theta on most X-ray diffractometers under standard conditions, that is when measured at a temperature in the range of from 15 to 25 °C with Cu-Kalpha_{1,2} radiation having a wavelength of 0.15419 nm. Furthermore, a person skilled in the art will appreciate that relative peak intensities will show inter-apparatus variability as well as variability due to degree of crystallinity, preferred orientation, sample preparation and other factors known to those skilled in the art and should be taken as qualitative measure only.

In the context of the present invention a reference to X-ray powder diffraction patterns means that they were measured / are to be measured at a temperature in the range of from 15 to 25 °C with Cu-Kalpha_{1,2} radiation having a wavelength of 0.15419 nm.

As used herein, the terms "essentially pure" or "substantially pure" with reference to a physical form of selexipag, means that the physical form includes less than about 10%, more preferably less than about 5%, even more preferably less than about 3%, and most preferably less than about 1% by weight of any other physical form of selexipag.

The term "physical form" as used herein refers to any crystalline and amorphous phase of selexipag.

The term "about" as used herein means within 5%, more typically within 1% and most typically within 0.5% of the indicated value or range.

As used herein, the term "mother liquor" refers to the solution remaining after crystallization of a solid.

### Abbreviations

- PXRD: powder X-ray diffraction
- DSC: differential scanning calorimetry
- TGA: thermogravimetric analysis
- GMS: gravimetric moisture sorption
- RH: relative humidity
- Tg: glass transition temperature

### BRIEF DESCRIPTION OF THE DRAWINGS

- Figure 1:: Representative PXRD of amorphous selexipag prepared according to the procedure described in Comparative Example 1 herein. The X-axis shows the 2-theta angle / °, with tick marks, from left to right, at 10, 20, 30 ° 2-theta. The Y-axis shows the intensity / counts, with tick marks, from bottom to top, at 500, 1000.
- Figure 2:: Representative PXRD of amorphous selexipag prepared according to the procedure described in comparative example 1 herein, after 6 weeks at 40 °C and 75% RH. The X-axis shows the 2-theta angle / °, with tick marks, from left to right, at 10, 20, 30 ° 2-theta. The Y-axis shows the intensity / counts, with tick marks, from bottom to top, at 500, 1000.

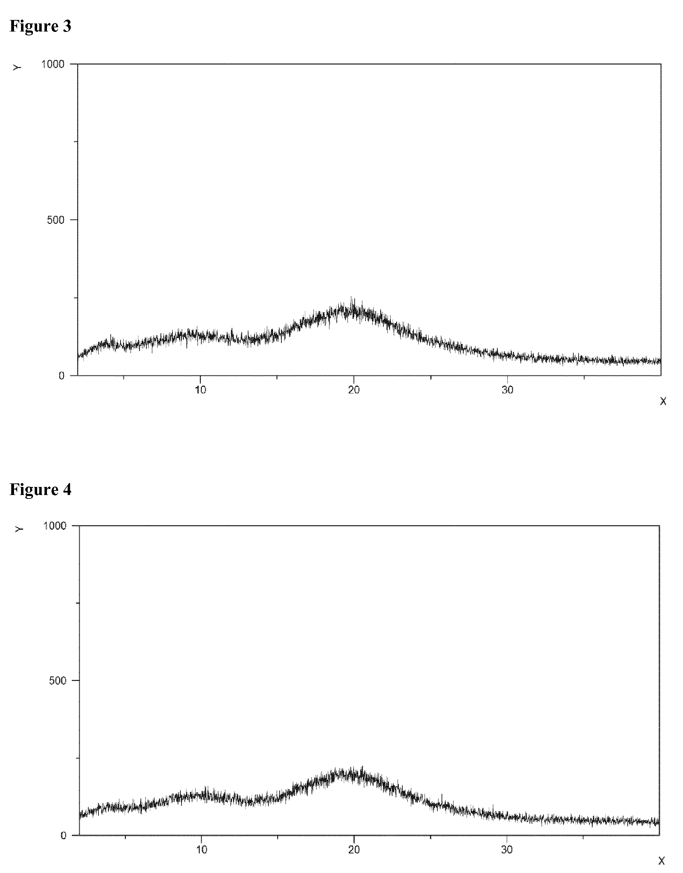

- Figure 3:: Representative PXRD of amorphous solid dispersion of selexipag and HPMCAS prepared according to the procedure described in Example 1 herein.The X-axis shows the 2-theta angle / °, with tick marks, from left to right, at 10, 20, 30 ° 2-theta. The Y-axis shows the intensity / counts, with tick marks, from bottom to top, at 500, 1000.
- Figure 4:: Representative PXRD of amorphous solid dispersion of selexipag and HPMCAS after storage at 40 °C and 75% relative humidity for 6 weeks. The X-axis shows the 2-theta angle / °, with tick marks, from left to right, at 10, 20, 30 ° 2-theta. The Y-axis shows the intensity / counts, with tick marks, from bottom to top, at 500, 1000.

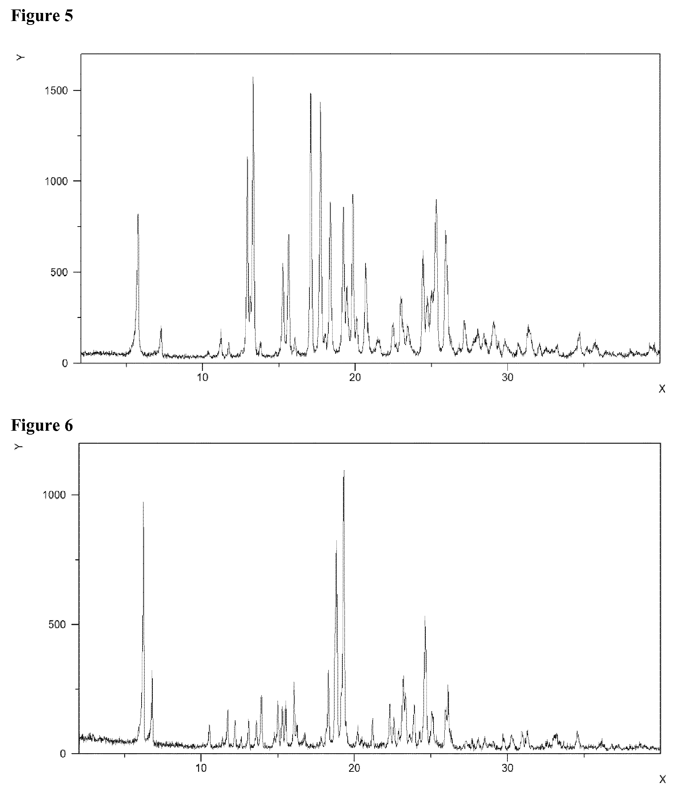

- Figure 5:: Representative PXRD of selexipag *o*-xylene solvate prepared according to Example 2 herein. The X-axis shows the 2-theta angle / °, with tick marks, from left to right, at 10, 20, 30 ° 2-theta. The Y-axis shows the intensity / counts, with tick marks, from bottom to top, at 500, 1000, 1500.
- Figure 6:: Representative PXRD of selexipag chlorobenzene solvate prepared according to Example 3.1 herein. The X-axis shows the 2-theta angle / °, with tick marks, from left to right, at 10, 20, 30 ° 2-theta. The Y-axis shows the intensity / counts, with tick marks, from bottom to top, at 500, 1000.

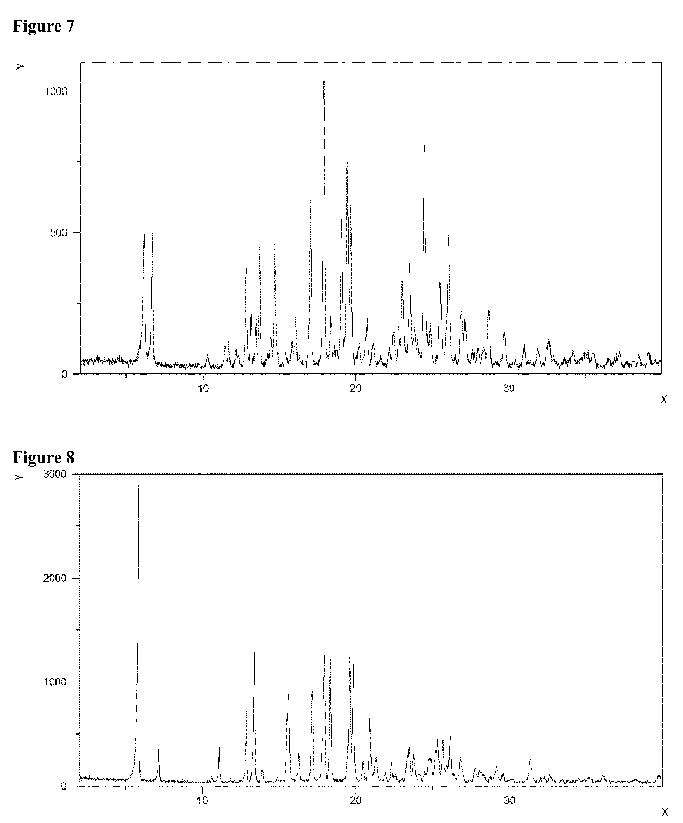

- Figure 7:: Representative PXRD of selexipag chlorobenzene solvate prepared according to Example 3.2 herein. The X-axis shows the 2-theta angle / °, with tick marks, from left to right, at 10, 20, 30 ° 2-theta. The Y-axis shows the intensity / counts, with tick marks, from bottom to top, at 500, 1000.
- Figure 8:: Representative PXRD of selexipag toluene solvate prepared according to Example 4 herein. The X-axis shows the 2-theta angle / °, with tick marks, from left to right, at 10, 20, 30 ° 2-theta. The Y-axis shows the intensity / counts, with tick marks, from bottom to top, at 1000, 2000, 3000.

### DETAILED DESCRIPTION OF THE INVENTION

When the invention is described below in further detail by reference to embodiments, it is not limited thereto.

In one aspect, the present invention relates to a solid dispersion comprising amorphous selexipag and at least one pharmaceutically acceptable excipient, wherein the at least one pharmaceutically acceptable excipient is (i) a polymer, or (ii) a silicon-based inorganic adsorbent.

The absence of peaks in the powder X-ray diffractogram of the solid dispersion of the invention, in particularly the absence of characteristic PXRD peaks of forms I, II and III of selexipag, suggests that in the solid dispersion of the present invention selexipag is substantially in amorphous form.

The characteristic powder X-ray diffraction pattern of selexipag form I comprises peaks at 2-theta angles of 9.4 ± 0.2°, 9.8 ± 0.2°, 17.2 ± 0.2° and 19.4 ± 0.2°. The characteristic X-ray powder diffraction pattern of selexipag form II comprises peaks at 2-theta angles of 9,0 ± 0.2°, 12.9 ± 0.2°, 20.7 ± 0.2° and 22.6 ± 0.2°. The characteristic X-ray powder diffraction pattern of selexipag form III comprises peaks at 2-theta angles of 9.3 ± 0.2°, 9.7 ± 0.2°, 16.8 ± 0.2°, 20.6 ± 0.2°, and 23.5 ± 0.2°.

The present invention circumvents the drawback of the known crystalline forms of selexipag, i.e. forms I, II and III, by providing a solid dispersion comprising amorphous selexipag and at least one pharmaceutically acceptable excipient as matrix material, wherein the at least one pharmaceutically acceptable excipient is (i) a polymer, or (ii) a silicon-based inorganic adsorbent. The solid dispersion of the present invention is obtainable as a solid dispersion that is stable and pure with regard to the physical form of amorphous selexipag.

The selexipag in the context of the solid dispersion of the present invention is stable in the amorphous state upon storage, as judged by the absence of characteristic PXRD peaks after storage. Amorphous selexipag itself readily converts to a mixture comprising selexipag form II and selexipag form III upon storage for as short as one week only.

"Stable in the amorphous stage upon storage" within the meaning of the present invention means that the solid dispersion of the present invention after storage at a relative humidity of 45% at 23°C for 14 days, preferably even after storage for 50 days, shows no signs of the presence of crystalline selexipag as judged by the absence of peaks at 2-theta angles of 9,0 ± 0.2°, 12.9 ± 0.2°, 20.7 ± 0.2° and 22.6 ± 0.2° or as judged by the absence of peaks at 2-theta angles of 9.3 ± 0.2°, 9.7 ± 0.2°, 16.8 ± 0.2°, 20.6 ± 0.2°, and 23.5 ± 0.2°. In particular the solid dispersion of the present invention after storage at a relative humidity of 45% at 23°C for 14 days shows no signs of the presence of any one of the known forms I, II and III of crystalline selexipag, as judged by the absence of peaks at 2-theta angles of 9.4 ± 0.2°, 9.8 ± 0.2°, 17.2 ± 0.2° and 19.4 ± 0.2° (form I), 9.0 ± 0.2°, 12.9 ± 0.2°, 20.7 ± 0.2° and 22.6 ± 0.2° (form II), and 9.3 ± 0.2°, 9.7 ± 0.2°, 16.8 ± 0.2°, 20.6 ± 0.2°, and 23.5 ± 0.2° (form III) in an powder X-ray diffractogram (PXRD).

"Stable in the amorphous state upon storage under stress conditions" in the context of the present invention means that the solid dispersion of the present invention when stored at a relative humidity of 70 % at 40 °C for 14 days, preferably after storage for 50 days, shows no signs of the presence of crystalline selexipag as judged by the absence of peaks at 2-theta angles of 9,0 ± 0.2°, 12.9 ± 0.2°, 20.7 ± 0.2° and 22.6 ± 0.2°, or as judged by the absence of peaks at 2-theta angles of 9.3 ± 0.2°, 9.7 ± 0.2°, 16.8 ± 0.2°, 20.6 ± 0.2°, and 23.5 ± 0.2°, and in particular shows no signs of the presence of any one of the known forms I, II and III of crystalline selexipag, as judged by the absence of peaks at 2-theta angles of 9.4 ± 0.2°, 9.8 ± 0.2°, 17.2 ± 0.2° and 19.4 ± 0.2° (form I), 9.0 ± 0.2°, 12.9 ± 0.2°, 20.7 ± 0.2° and 22.6 ± 0.2° (form II), and 9.3 ± 0.2°, 9.7 ± 0.2°, 16.8 ± 0.2°, 20.6 ± 0.2°, and 23.5 ± 0.2° (form III) in an powder X-ray diffractogram (PXRD).

Thus, in a preferred embodiment, the solid dispersion of the present invention is a solid dispersion wherein the amorphous selexipag is stable in the amorphous physical form upon storage. More preferably, the solid dispersion of the present invention is a solid dispersion wherein the amorphous selexipag is stable in the amorphous physical form upon storage under stress conditions.

It is preferred that the solid dispersion of the invention itself is amorphous, i.e. that the solid dispersion of the present invention does not only show no sign of the presence of any crystalline selexipag, but that the solid dispersion comprising amorphous selexipag and at least one pharmaceutically acceptable excipient, wherein the at least one pharmaceutically acceptable excipient is (i) a polymer, or (ii) a silicon-based inorganic adsorbent, shows no characteristic peak, preferably no peak, in an PXRD. It is more preferred that the amorphous solid dispersion is stable in the amorphous physical form upon storage, and in particular upon storage under stress conditions.

In one aspect, the pharmaceutically acceptable excipient is an organic polymer, preferably a hydrophilic organic polymer. Preferably, the at least one organic polymer is a homopolymer and/or copolymers. Polymers that contain only a single type of repeat unit are known as homopolymers, while polymers containing a mixture of repeat units are known as copolymers.

Examples of hydrophilic organic polymers include, but are not restricted to, polysaccharides, preferably cellulose derivatives, polyvinylpyrrolidones, polyethylene glycols, polyethylene glycol based copolymers, polyacrylic acids, salts of polyacrylic acids, polyvinyl alcohols, polyacrylamide copolymers, methacrylic acid copolymers, methacrylate copolymers, pectines, chitin derivatives, chitosan derivatives, polyphosphates, polyoxazolines, polyvinyl caprolactam-polyvinyl acetate-polyethylene glycol copolymers, and mixtures of two or more thereof. More specific examples of hydrophilic polymers include, but are not restricted to, cellulose derivatives selected from the group consisting of alkylcellulose, preferably methylcellulose, ethylcellulose, or propylcellulose; hydroxyalkylcellulose, preferably hydroxymethylcellulose, hydroxyethylcellulose, or hydroxypropylcellulose; hydroxyalkylalkylcellulose, preferably hydroxyethylmethylcellulose (HEMC), or hydroxypropylmethylcellulose (HPMC); carboxyalkylcellulose, preferably carboxymethylcellulose (CMC), carboxymethylhydroxyethylcellulose (CMHEC), hydroxyethylcarboxymethylcellulose (HECMC); sodium carboxymethylcellulose, cellulose acetate phthalate (CAP), hydroxypropylmethylcellulose acetate (HPMCA), hydroxypropylmethylcellulose phthalate (HPMCP), hydroxypropylmethylcellulose acetate succinate (HPMCAS), and a mixture of two or more thereof. In case the polymer is a polyvinylcaprolactam-polyvinylacetate-polyethyleneglycol copolymer, the polymer is preferably a graft polymer. The term "graft polymer" as used herein refers to a branched copolymer in which the side chains are structurally distinct from the main chain. Preferably, these graft polymers are based on polyethers and are obtained via free radical polymerization of the vinyl monomers in the presence of polyethers. In case of a polyvinylcaprolactam polyvinylacetate polyethyleneglycol graft polymer, a vinylcaprolactam and vinylacetate are used as vinyl monomers. Graft polymers of this type are commercially available as Soluplus^{®} from BASF.

Hydroxypropylmethylcellulose acetate succinate (HPMCAS) and polyethyleneglycolpolyvinyl caprolactam- polyvinyl acetate grafted copolymer are particularly preferred.

In another aspect, the pharmaceutically acceptable excipient is a silicon-based inorganic compound.

Examples of silicon-based inorganic compounds include, but are not restricted to, silica, silicates, and a combination of two or more thereof. For example, the silicon-based inorganic adsorbent is selected from the group consisting of silicas and combinations of two or more thereof; or from the group consisting of silicates and combinations of two or more thereof; or from the group consisting of at least one silica and at least one silicate. The term "silicate" as used in this context of the present invention refers to naturally occurring or synthesized compounds containing an anionic silicon compound, preferably an oxide. Examples of such silicates include, but are not restricted to, nesosilicates comprising the structure unit [SiO₄]⁴⁻, sorosilicates comprising the structure unit [Si₂O₇]⁶⁻, cyclosilicates comprising the structure unit [SiₙO_{3n]}²ⁿ⁻, single chain inosilicates comprising the structure unit [SiₙO₃ₙ]²ⁿ⁻, double chain inosilicates comprising the structure unit [Si₄ₙO₁₁ₙ]⁶ⁿ⁻, phyllosilicates comprising the structure unit [SiₙO₅ₙ]²ⁿ⁻, or tectosilicates with a 3D framework comprising the structure unit [AlₓSi_{y}O_{2(x+y)}]^{x-}. The term "silica" as used in this context of the present invention refers to naturally occurring or synthesized silica. Examples of such silica include, but are not restricted to fumed silica, precipitated silica, gel silica, colloidal silica.

Silicon-based inorganic compounds are preferred which have a pH in a defined range, preferably a pH of at least 6.0. More preferably, the at least one silicon-based compound has a pH in the range of from 6.0 to 9.0, more preferably in the range of from 6.5 to 8.5, more preferably in the range of from 7.0 to 8.0.

Generally, it is conceivable that the solid dispersion of the present invention contains at least one silicon-based inorganic adsorbent having a pH in the above-defined preferred ranges and at least one silicon-based inorganic adsorbent having a pH outside these ranges. Preferably, all silicon-based inorganic adsorbents comprised in the solid composition of the present invention have a pH in the above-defined preferred ranges.

The solid dispersion according to the present invention can also comprise at least one hydrophilic, organic polymer and at least one silicon-based inorganic compound. Generally, it is possible that the solid dispersion contains at least one hydrophilic, organic polymer and at least one silicon-based inorganic adsorbent.

For the solid dispersion of the invention comprising amorphous selexipag and at least one pharmaceutically acceptable excipient, the weight ratio of selexipag and the pharmaceutically acceptable excipient is from 1.0 : 0.5 to 1.0 : 8.0, such as 1.0 : 0.5 to 1.0 : 5.0, for example 1.0 : 0.5 to 1.0 : 3.0. The weight ratio is calculated based on the total amount of selexipag present in said solid dispersion and based on the total amount of the pharmaceutically acceptable excipients present in said solid dispersion, i.e. if two or more pharmaceutically acceptable excipients are present in the solid dispersion of the present invention, the combined weight of these is taken for the calculation of the weight ratio. A preferred weight ratio is from 1.0 : 0.5 to 1.0 : 2.0.

In a further preferred embodiment, the solid dispersion of the present invention is a solid dispersion wherein amorphous selexipag is chemically stable.

By "chemically stable" it is meant that amorphous selexipag present in the solid dispersion of the present invention shows very little degradation upon storage under stress conditions, i.e. when stored at a relative humidity of 70 % at 40°C for 14 days, preferably after storage for 50 days. Very little degradation means that an HPLC analysis of selexipag shows no impurity of more than 0.1 area% under the following analysis conditions: sample concentration of 1mg/mL in isopropanol or ethanol, CHIRALPAK AD-H (250 x 4,6 mm) column, flow of solvent A and solvent B (90:10) of 1.0 mL/min with hexane as solvent A and hexane/ethanol/butylamine (90:10:0.02) as solvent B, column temperature of 10 °C and wavelength of 226 nm.

### First preferred embodiment

According to a first preferred embodiment of the present invention, the at least one pharmaceutically acceptable excipient used as matrix component is at least one hydrophilic, polymer, preferably consists of at least one, more preferably one, hydrophilic, organic polymer.

Therefore, the present invention also relates to a solid dispersion comprising amorphous selexipag according to formula (I) and at least one pharmaceutically acceptable excipient wherein at least 99 weight-% of the selexipag comprised in the solid dispersion are present in amorphous form, at least 99 weight-% of the solid dispersion consist of the selexipag and the at least one pharmaceutically acceptable excipient, and wherein the at least one pharmaceutically acceptable excipient is a hydrophilic, organic polymer.

Examples of hydrophilic, organic polymers include, but are not restricted to, polysaccharides, preferably cellulose derivatives, polyvinylpyrrolidones, polyethylene glycols, polyethylene glycol based copolymers, polyacrylic acids, salts of polyacrylic acids, polyvinyl alcohols, polyacrylamide copolymers, methacrylic acid copolymers, methacrylate copolymers, pectines, chitin derivatives, chitosan derivatives, polyphosphates, polyoxazolines, polyvinyl caprolactam-polyvinyl acetate-polyethylene glycol copolymers, and mixtures or combinations of two or more thereof.

With regard to the chemical nature of the at least one hydrophilic, organic polymer, in one aspect polysaccharides and derivatives of polysaccharides are preferred. The polysaccharides can be homoglycans or heteroglycans. Further, the polysaccharides can be naturally occurring compounds or synthesized compounds. Regarding the derivatives of polysaccharides, compounds are preferred which are derivatized at one or more hydroxyl groups of the monosaccharide units of the polysaccharides. Polysaccharides and derivatives of polysaccharides include, but are not restricted to, cellulose and cellulose derivatives, such as alkylcellulose, such as methylcellulose, ethylcellulose, or propylcellulose; hydroxyalkylcellulose, such as hydroxymethylcellulose, hydroxyethylcellulose, or hydroxypropylcellulose; hydroxyalkylalkylcellulose, such as hydroxyethylmethylcellulose (HEMC), or hydroxypropylmethylcellulose (HPMC); carboxyalkylcellulose, such as carboxymethylcellulose (CMC), carboxymethylhydroxyethylcellulose (CMHEC), hydroxyethylcarboxymethylcellulose (HECMC); sodium carboxymethylcellulose, cellulose acetate phthalate (CAP), hydroxypropylmethylcellulose acetate (HPMCA), hydroxypropylmethylcellulose phthalate (HPMCP), hydroxypropylmethylcellulose acetate succinate (HPMCAS), and a mixture or combination of two or more thereof. Preferably, the at least one hydrophilic, organic polymer comprises, preferably consists of a cellulose derivative selected from the group consisting of hydroxyalkylalkylcelluloses derivatives and a mixture of two or more thereof. More preferably, the at least one hydrophilic, polymer comprises, more preferably consists of, hydroxypropylmethylcellulose acetate succinate (HPMCAS).

Preferably, the average molecular weight (M_{w}) of the cellulose derivative, preferably the hydroxyalkylalkylcellulose derivative, more preferably the hydroxypropylmethylcellulose acetate succinate (HPMCAS), is in the range of from 7 to 225 kDa, more preferably in the range of from 7 to 100 kDa, more preferably in the range of from 7 to 30 kDa. According to the present invention, it is possible that the solid composition contains two or more cellulose derivative, preferably two or more hydroxyalkylalkylcellulose derivatives, more preferably two or more hydroxypropylmethylcellulose derivatives, which differ only in the average molecular weight M_{w}.

Preferably, the degree of substitution of methoxy groups (DS_{M}) of the cellulose derivative, preferably the hydroxyalkylalkylcellulose derivative, more preferably the hydroxypropylmethylcellulose acetate succinate (HPMCAS), is in the range of from 0.3 to 2.8, more preferably in the range of from 0.6 to 2.5, more preferably in the range of from 1.0 to 2.3, more preferably in the range of from 1.3 to 2.0. According to the present invention, it is possible that the solid composition contains two or more cellulose derivative, preferably two or more hydroxyalkylalkylcellulose derivatives, more preferably two or more hydroxypropylmethylcellulose derivatives, which differ only in the degree of substitution. The parameter DS_{M} describes the average number of methoxy substitutions per anhydroglucose unit of a given hydroxyalkylalkylcellulose derivative.

Further according to the present invention, it is possible that the solid dispersion contains two or more cellulose derivatives, preferably two or more hydroxyalkylalkylcellulose derivatives, more preferably two or more hydroxypropylmethylcellulose derivatives, which differ in the degree of substitution of methoxy groups and the average molecular weight M_{w}.

Also, the present invention also relates to a solid dispersion comprising amorphous selexipag according to formula (I) and at least one pharmaceutically acceptable excipient, wherein at least 99 weight-% of the selexipag comprised in the solid dispersion are present in amorphous form, at least 99 weight-% of the solid dispersion consist of the selexipag and the at least one pharmaceutically acceptable excipient, and wherein the at least one pharmaceutically acceptable excipient is a cellulose derivative selected from the group consisting of hydroxyalkylalkylcellulose derivatives and a mixture of two or more thereof, the cellulose derivative preferably comprising, more preferably consisting of, hydroxypropylmethylcellulose acetate succinate (HPMCAS).

With regard to the chemical nature of the at least one hydrophilic, organic polymer, in another aspect polyethyleneglycol- polyvinyl caprolactam- polyvinyl acetate grafted copolymer is preferred. Polyethyleneglycol- polyvinyl caprolactam- polyvinyl acetate grafted copolymer is commercially available as Soloplus^{®} from BASF.

### Second preferred embodiment

Therefore, according to a second preferred embodiment of the present invention, the at least one pharmaceutically acceptable excipient used as matrix component is a silicon-based inorganic compound, preferably consists of at least one, more preferably one silicon-based inorganic compound.

Therefore, the present invention also relates to a solid dispersion comprising amorphous selexipag according to formula (I) and at least one pharmaceutically acceptable excipient, wherein the at least one pharmaceutically acceptable excipient is a silicon-based inorganic compound, and wherein at least 99 weight-% of the selexipag comprised in the solid dispersion are present in amorphous form, at least 99 weight-% of the solid dispersion consist of the selexipag and the at least one silicon-based inorganic compound.

Also, the present invention also relates to a solid composition comprising amorphous selexipag according to formula (I) and at least one pharmaceutically acceptable excipient, wherein the at least one pharmaceutically acceptable excipient is a silicon-based inorganic compound, and wherein at least 99 weight-% of the selexipag comprised in the solid dispersion are present in amorphous form, at least 99 weight-% of the solid dispersion consist of the selexipag and the at least one silicon-based inorganic compound, and wherein the pH of the silicon-based inorganic compound is in the range of from 6.0 to 9.0, preferably in the range of from 6.5 to 8.5, more preferably in the range of from 7.0 to 8.0.

Preferably, the bulk density of the at least one silicon-based inorganic compound is in the range of from 10 to 500 g/ml, preferably in the range of from 30 to 400 g/ml, more preferably in the range of from 50 to 300 g/ml. Generally, it is conceivable that the solid dispersion of the present invention contains at least one silicon-based inorganic compound having a bulk density in the above-defined preferred ranges and at least one silicon-based inorganic compound having a bulk density outside these ranges. Preferably, all silicon-based inorganic compounds comprised in the solid composition of the present invention have a bulk density in the above-defined preferred ranges.

In one aspect, the silicon-based inorganic compound is silica. The silica is preferably selected from the group consisting of fumed silica, precipitated silica, gel silica, colloidal silica, and a combination of two or more thereof, such as a combination of fumed silica and precipitated silica or a combination of fumed silica and colloidal silica or a combination of fumed silica and gel silica or a combination of precipitated silica and gel silica or a combination of precipitated silica and colloidal silica or a combination of gel silica and colloidal silica or a combination of fumed silica and precipitated silica and gel silica or a combination or fumed silica and gel silica and colloidal silica or a combination of precipitated silica and gel silica and colloidal silica or a combination of fumed silica and precipitated silica and gel silica and colloidal silica. Preferred silica include, but are not restricted to, the commercially available compounds Syloid^{®} 72 FP, Syloid^{®} 244 FP, both from Grace.

In another aspect, the silicon-based compound is a silicate. The silicate is preferably an aluminosilicate which, more preferably, additionally contains at least one alkali metal element selected from the group consisting of Li, Na, K, Rb, Cs and a combination of two or more thereof, preferably from the group consisting of Li, Na, K, and a combination of two or more thereof, more preferably from the group consisting of Na, K, and a combination of two or more thereof, and/or at least one alkaline earth metal element selected from the group consisting of Mg, Ca, Sr, Ba, and a combination of two or more thereof, preferably from the group consisting of Mg, Ca, Ba, and a combination of two or more thereof, preferably from the group consisting of Mg, Ca, and a combination of two or more thereof. More preferably, the silicate is an aluminosilicate which additionally contains at least one alkaline earth metal element selected from the group consisting of Mg, Ca, Sr, Ba, and a combination of two or more thereof, preferably from the group consisting of Mg, Ca, Ba, and a combination of two or more thereof, preferably from the group consisting of Mg, Ca, and a combination of two or more thereof. More preferably, the silicate is an aluminosilicate, which additionally contains Mg. Preferred silicates include, but are not restricted to, the commercially available compounds Neusilin^{®} UFL2, Neusilin^{®} US2, both from Fuji Chemical Industry Co., Ltd.

Therefore, the present invention also relates to the solid dispersion as described above, wherein the at least one silicon-based inorganic compound is selected from the group consisting of silica, silicates, and a combination of two or more thereof, wherein the silica is preferably selected from the group consisting of fumed silica, precipitated silica, gel silica, colloidal silica, and a combination of two or more thereof, and wherein the silicates are preferably aluminosilicates, preferably comprising at least one alkali metal element and/or at least one alkaline earth metal element, more preferably at least one alkaline earth metal element, more preferably magnesium.

Generally, the silica and/or the silicate can be present in crystalline or amorphous form. Preferably, at least 90 weight-%, more preferably at least 95 weight-%, more preferably at least 99 weight-% of the at least one silicon-based inorganic compound are present in amorphous form. More preferably, at least 99.5 weight-%, more preferably at least 99.9 weight-%, more preferably at least 99.99 weight-% of the at least one silicon-based inorganic compound are present in amorphous form.

### Preparation process of the solid dispersion

As described above, the present invention also relates to a process for preparing a solid dispersion comprising amorphous selexipag and at least one pharmaceutically acceptable excipient, wherein the at least one pharmaceutically acceptable excipient is (i) a polymer, or (ii) a silicon-based inorganic adsorbent, the process comprising:
a) providing selexipag;
b) dissolving or dispersing selexipag provided in (a) and the at least one pharmaceutically acceptable excipient, wherein the at least one pharmaceutically acceptable excipient is (i) a polymer, or (ii) a silicon-based inorganic adsorbent,
   in a solvent to form a mixture; and
c) removing at least part, preferably essentially all, of the solvent to provide the solid dispersion.

### Step a)

Selexipag may be provided by any method known to the person skilled in the art, such as, e.g., the methods described in EP 2447254 A1.

Selexipag may be provided in any form, such as in crystalline, in amorphous form, or in a mixture of thereof.

Preferably, selexipag in step (a) is provided in amorphous form such as described in EP 2447254 A1, table 4, solvent 30.

However, selexipag provided in step (a) may also be present in crystalline form I, II or III or as a mixture of two or more thereof, for example as a mixture of form II and form III.

Preferably, selexipag is provided as a crystalline solvate with a solvation partner, which is an aromatic hydrocarbon, preferably selected from toluene, chlorobenzene and o-xylene. A crystalline solvate of selexipag and an aromatic hydrocarbon is thus a preferred intermediate for the production of the solid dispersion of the present invention. In particular, a toluene solvate of selexipag is a preferred intermediate for the production of the solid dispersion of the present invention. Also, a crystalline chlorobenzene solvate of selexipag is a preferred intermediate for the production of the solid dispersion of the present invention. Also, a crystalline o-xylene solvate of selexipag is a preferred intermediate for the production of the solid dispersion of the present invention. The invention therefore also relates to these solvates of selexipag with aromatic hydrocarbons. The invention also relates to the preparation of solvates of selexipag with aromatic hydrocarbons. The invention further relates to the use of crystalline solvates of selexipag with aromatic hydrocarbons for the preparation of amorphous selexipag.

Thus, the present invention also relates to a process for preparing a solid dispersion and a solid dispersion obtained or obtainable by the above-described method, wherein in step (a) crystalline selexipag, preferably a crystalline solvate of selexipag with aromatic hydrocarbons or crystalline selexipag in polymorphic form I or II or III, or a mixture of two or more of these crystalline forms, more preferably crystalline selexipag in polymorphic form II or III or as a mixture of thereof, is provided.

Alternatively, selexipag in step (a) is provided in amorphous form.

### Step (b)

In step (b), selexipag is preferably dissolved or dispersed together with the at least one pharmaceutically acceptable excipient as matrix compound in a suitable solvent. As excipient (i) a polymer, or (ii) a silicon-based inorganic adsorbent can be used. Both components may be dissolved or dispersed together or subsequently.

The term "a suitable solvent" as used herein refers to a solvent or solvent mixture in which both selexipag and the at least one pharmaceutically acceptable excipient have adequate solubility or may be suitably dispersed. The term "adequate solubility" is denoted to mean a solubility at room temperature of selexipag of greater than about 10 mg/ml.

In the case where the pharmaceutically acceptable excipient is an organic polymer, preferably a hydrophilic organic polymer, selexipag and the organic polymer may require different solvents to obtain the desired solubility. A mixture of solvents can then be used. In this case, selexipag may be dissolved in at least one solvent to give a mixture comprising the at least one solvent and selexipag. Likewise, the organic polymer may be dissolved in at least one further solvent to give a mixture comprising the polymer and the at least one further solvent. Both mixtures may then be mixed together.

Selexipag and organic polymer may be dissolved or dispersed, together or subsequently, in the suitable solvent (including solvent mixtures).

Examples of suitable solvents include, but are not limited to, water, C1-C3 ketones, C1-C2 halogenated hydrocarbons, C3-C4 alcohols, C2-C6 ethers, C3-C5 esters, and a combination of two or more thereof, more preferably from the group consisting of C1-C3 ketones, and a combination of two or more thereof. More preferably, the solvent comprises, and for example consists of, dichloromethane, chloroform, ethanol, methanol, THF, methyl THF, 2-propanol, ethyl acetate, acetone, water or mixtures of two or more thereof. Preferably, the solvent comprises, and for example consists of, dichloromethane, THF, methyl THF, acetone, or mixtures of two or more thereof.

The solution obtained in step (b) may be directly used in step (c) of the method according to the invention. According to a preferred embodiment, the solution formed is purified before used in step (c). The term "purified" in this context means e.g. that non-dissolved particles, such as non-dissolved organic polymer and/or non-dissolved selexipag, may be removed by suitable methods known to those skilled in the art such as centrifugation, filtration, ultrafiltration or the like. Preferably, the solution in step (b) is filtrated prior to step (c).

Thus, the present invention also relates to a process, as described above, the process comprising
(a) providing selexipag,
(b) dissolving selexipag provided in step (a) and the at least one organic polymer in a solvent to form a solution, then optionally filtrating the solution, and
(c) removing at least part, preferably essentially all, of the solvent
to give the solid dispersion.

To accelerate and/or improve the dissolution process of selexipag in the at least one solvent, suitable methods can be applied. For example, the dissolution process can be influenced by choosing suitable temperatures, by stirring, and/or by subjecting the respective mixtures to sonication, wherein these methods can be applied during the entire or one or more parts of the mixing process.

Preferably, the solution of selexipag and the at least one organic polymer in the at least one solvent, is prepared at a temperature in the range of from 10 to 40 °C, more preferably in the range of from 15 to 35 °C, more preferably in the range of from 20 to 30 °C, preferably at ambient pressure.

In the case where pharmaceutically acceptable excipient is a silicon-based inorganic compound, it is preferred that the process comprises dispersing the at least one silicon-based inorganic compound in a solution comprising the dissolved selexipag.

Consequently, solvents are preferred in which selexipag can be dissolved and the at least one silicon-based inorganic compound can be dispersed. Preferably, the at least one suitable solvent is selected from the group consisting of water, C1-C3 ketones, C1-C2 halogenated hydrocarbons, C3-C4 alcohols, C2-C6 ethers, C3-C5 esters, and a combination of two or more thereof, more preferably from the group consisting of C1-C3 ketones or C1-C2 halogenated hydrocarbons, and a combination of two or more thereof. Preferably, the solvent comprise dichloromethane or acetone. More preferably, the solvent is dichloromethane or acetone.

Regarding the weight ratio of selexipag and the at least one silicon-based inorganic compound relative to the at least one solvent, no specific restrictions exist provided that the finally obtained mixture is a mixture wherein the at least one silicon-based inorganic compound is dispersed in a solution of the selexipag in the at least one solvent, which mixture can be subjected to the subsequent step (c). Preferably, the weight ratio of selexipag plus the at least one silicon-based inorganic compound, preferably selexipag plus the at least one silica, relative to the at least one solvent, preferably dichloromethane or acetone, is in the range of from 0.01 : 1 to 0.4 : 1, preferably in the range of from 0.02 : 1 to 0.3 : 1, more preferably in the range of from 0.05 : 1 to 0.3 : 1. Also preferably, the weight ratio of selexipag plus the at least one silicon-based inorganic compound, preferably selexipag plus the at least one silicate, preferably the aluminosilicates preferably comprising at least one alkali metal element and/or at least one alkaline earth metal element, more preferably at least one alkaline earth metal element, more preferably magnesium, relative to the at least one solvent, preferably dichloromethane or acetone, is in the range of from 0.01 : 1 to 1.4 : 1, preferably in the range of from 0.02 : 1 to 0.3 : 1, more preferably in the range of from 0.05 : 1 to 0.3 : 1.

To accelerate and/or improve the dissolution process of selexipag in the at least one solvent, suitable methods can be applied. For example, the dissolution process can be influenced by choosing suitable temperatures, by stirring, and/or by subjecting the respective mixtures to sonication, wherein these methods can be applied during the entire or one or more parts of the mixing process.

Preferably, the dispersion of the at least one silicon-based inorganic compound, preferably selected from the group consisting of silica, silicates, and a combination of two or more thereof, in the solution of selexipag in the at least one solvent, is prepared at a temperature in the range of from 10 to 40 °C, more preferably in the range of from 15 to 35 °C, more preferably in the range of from 20 to 30 °C, preferably at ambient pressure.

### Step (c)

In step (c) of the above described method, at least part, preferably essentially all, of the solvent is removed. "Essentially all" is denoted to mean that at least 95 % by weight, more preferably at least 96 % by weight, more preferably at least 97 % by weight, more preferably at least 98 % by weight, more preferably at least 99 % by weight, more preferably at least 99.9 % by weight, more preferably all of the solvent present in the solution according to step (b) is removed in step (c).

Preferably, the solid dispersion obtained or obtainable by this process thus comprises less than 5 % by weight, more preferably less than 4 % by weight, more preferably less than 3 % by weight, more preferably less than 2 % by weight, more preferably less than 1 % by weight, more preferably less than 0.1 % by weight, based on the total weight of the solid dispersion, of the solvent. Most preferably, all of the solvent present in the solution is removed to give the solid dispersion.

The removal of the solvent may be carried out by any suitable method known to those skilled in the art such as evaporation, spray drying, lyophilization, melt extrusion, drum drying, freeze-drying or other solvent removal processes. Preferably, the solvent is removed by spray drying or evaporation. Spray drying is a process well known to those skilled in the art for preparing solid dispersions. In such a spray drying process, the solution is pumped through an atomizer into a drying chamber thereby removing the solvent to form the solid dispersion. A drying process uses hot gases, such as air, nitrogen, nitrogen-enriched air or argon, to dry the particles. The solution can be atomized by conventional means well known in the art, such as a two-fluid sonication nozzle and a two-fluid non-sonication nozzle.

Preferably, the solvent is removed by evaporation, such as by evaporation under reduced pressure. Preferably, the pressure in step (c) is in the range of from 50 to 450 mbar, more preferably in the range of from 50 to 250 mbar, more preferably in the range of from 50 to 200 mbar.

The temperature during evaporation may be varied or held essentially constant and is preferably in the range of from 20 to 40 °C, more preferably in the range of from 25 to 40 °C, and most preferably in the range of from 30 to 40 °C.

### Pharmaceutical composition

In another aspect of the invention, the present invention relates to a pharmaceutical composition comprising a solid dispersion as described above or a solid dispersion obtained or obtainable by the above-described method. The pharmaceutical composition preferably comprises the solid dispersion as key ingredient together with at least one further excipient in addition to the at least one matrix compound present in the solid dispersion. The at least one further excipient may be included in the solid dispersion or may be subsequently added to or mixed with the dispersion. Preferably, the pharmaceutical composition comprises no further selexipag in addition to the selexipag present in the solid dispersion. Thus, preferably, at least 80 % by weight, more preferably at least 85 % by weight, more preferably at least 90 % by weight , more preferably at least 95 % by weight, more preferably at least 96 % by weight , more preferably at least 97 % by weight, more preferably at least 98 % by weight, more preferably at least 99 % by weight, more preferably at least 99.9 % by weight, more preferably all of the selexipag present in the pharmaceutical composition is present in amorphous form.

Suitable pharmaceutical excipient include, but are not limited to, carriers, fillers, diluents, lubricants, sweeteners, stabilizing agents, solubilizing agents, antioxidants and preservatives, flavoring agents, binders, colorants, osmotic agents, preservatives, buffers, surfactants, granulating and disintegrating agents and combinations thereof.

Examples of diluents include, but are not limited to, calcium carbonate, sodium carbonate, lactose, calcium phosphate, microcrystalline cellulose, mannitol, starch, sodium phosphate or the like.

Examples for granulating and disintegrating agents are corn starch, alginic acid, sodium starch glycolate, crospovidone, croscarmellose sodium and the like.

The pharmaceutical composition of the present invention may optionally comprise one or more surfactants, which may be ionic or non-ionic surfactants. The surfactants can increase the rate of dissolution by facilitating wetting, thereby increasing the maximum concentration of dissolved drug. The surfactants may also facilitate the formulation process. Surfactants may also stabilize the amorphous dispersions by inhibiting crystallization or precipitation of the drug by interacting with the dissolved drug by such mechanisms as complexation, formation of inclusion complexes, formation of micelles, and adsorption to the surface of the solid drug. Suitable surfactants include cationic, anionic, and non-ionic surfactants.

These include for example fatty acids and alkyl sulfonates; cationic surfactants such as benzalkonium chloride; anionic surfactants, such as dioctyl sodium sulfosuccinate and sodium lauryl sulfate (sodium dodecyl sulfate); sorbitan fatty acid esters; Vitamin E TPGS; polyoxyethylene sorbitan fatty acid esters; polyoxyethylene castor oils and hydrogenated castor oils such as Cremophor RH-40 and Cremopher EL; Liposorb P-20, Capmul POE-0, and natural surfactants such as lecithin and other phospholipids and mono- and diglycerides.

Thus, the present invention also relates to a pharmaceutical composition as described above, wherein the composition comprises one or more surfactants selected from the group consisting of anionic surfactants and non-ionic surfactants.

Preferably, the pharmaceutical composition comprises one or more surfactants selected from sodium dodecyl sulfate and one or more non-ionic surfactants selected from (a) sorbitan fatty acid esters, (b) polyoxyethylene sorbitan fatty acid esters, (c) polyoxyethylene castor oils, (d) polyoxyethylene hydrogenated castor oils, and (e) vitamin E TPGS; and mixtures thereof.

Examples of carriers include, without limitation, solvents, saline, buffered saline, dextrose, water, glycerol, ethanol, propylene glycol, polysorbate 80 (Tween-80™), poly(ethylene)glycol 300 and 400 (PEG 300 and 400), PEGylated castor oil (e.g. Cremophor EL), poloxamer 407 and 188, hydrophobic carriers, fat emulsions, lipids, PEGylated phopholids, polymer matrices, biocompatible polymers, lipospheres, vesicles, particles, and liposomes, or combinations thereof.

Examples of osmotic agents include sodium chloride, glycerol, sorbitol, xylitol, glucose, or combinations thereof.

Binders can include acacia gum, starch, gelatin, sucrose, polyvinylpyrrolidone (Providone), sorbitol, tragacanth methylcellulose, sodium carboxymethylcellulose, hydroxypropyl cellulose, hydroxypropylmethylcellulose, ethylcellulose, or combinations thereof.

Examples of fillers can include calcium phosphate, glycine, lactose, maize-starch, sorbitol, sucrose, or combinations thereof.

Examples of lubricants include magnesium stearate or other metallic stearates, stearic acid, polyethylene glycol, waxes, oils, silica and colloical silica, silicon fluid, talc, or combinations thereof.

As flavoring agent those flavoring agents approved by the FDA for use in sweets and pharmaceuticals, foods, candies and beverages or the like are preferred. Preferably, these flavoring agents impart flavors such as grape, cherry, citrus, peach, strawberry, bubble gum, peppermint or others. Peppermint, methyl salicylate, orange flavoring and the like are mentioned by way of examples.

The pharmaceutical composition of the present invention preferably contains a therapeutically effective amount of selexipag. The term "therapeutically effective amount" as used herein refers to an amount of selexipag present in the solid dispersion or pharmaceutical composition being administered that is sufficient to prevent the development of, or alleviate to some extent one or more of the symptoms of the pulmonary arterial hypertension.

A pharmaceutical composition of the present invention can be a tablet, for example a tablet prepared from 1-5 mg solid dispersion, 80-120 mg lactose or mannitol as a filler suitable for direct tableting, 5-15 mg HPMC low viscosity or PVP25 as binder, 5-15 mg acdisol, primojel or PVPP as disintegrant, and 1-2 mg Mg stearate as lubricant, These ingredients may be mixed, tableted and optionally film-coated with a standard film known in the art.

The pharmaceutical composition of the present invention is preferably administered orally to patients, which include, but is not limited to, mammals, for example humans, in the form of, for example, a tablet, pills, granules or the like. It is also apparent that the pharmaceutical composition of the present invention can be administered with other therapeutic and/or prophylactic agents and/or medications that are not medically incompatible therewith.

The solid dispersion described above may be used in combination with other drugs that are used in the prevention, treatment, control, amelioration, or reduction of risk of the diseases or conditions for which compounds of the present invention are useful. Such other drugs may be administered, by a route and in an amount commonly used therefor, contemporaneously or sequentially with the solid dispersion of the present invention.

### Use

The solid dispersion, described above, or the pharmaceutical composition described above is useful in a method of providing an agonistic effect on the PGI₂ receptor. Thus, the present invention also describes the solid dispersion, described above, or a pharmaceutical composition, as described above for use as agonists of PGI₂ receptor activity. In particular, the solid dispersion, described above, or the pharmaceutical composition, described above is used for treating, preventing, ameliorating, controlling or reducing the risk of a variety of disorders associated with the PGI₂ receptor, in particular for the treatment of pulmonary hypertension, in particular pulmonary arterial hypertension.

In particular, the solid dispersion, described above, or the pharmaceutical composition, described above, is used for treating or preventing pulmonary hypertension, in particular pulmonary arterial hypertension.

As used herein, the terms "treatment" and "treating" refer to all processes wherein there may be a slowing, interrupting, arresting, controlling, or stopping of the progression of the disorders described herein, but does not necessarily indicate a total elimination of all disorder symptoms.

The dosage of selexipag in the compositions of this invention may be varied, however, it is necessary that the amount of selexipag be such that a suitable dosage form is obtained. The dosage regimen will be determined by the attending physician and other clinical factors. It is well known in the medical art that the dosage for anyone patient depends upon many factors including the patient's size, body surface area, age, sex, time and route of administration, general health and other drugs being administered concurrently. Efficacy can be monitored by periodic assessment. Selexipag may be administered to patients (animals and human) in need of such treatment in dosages that will provide optimal pharmaceutical efficacy. A typical dosage can be, for example, in the range of 100µg to 1600µg, such as 200µg, 300µg, 400µg, 500µg, 600µg, 700µg, 800µg, 900µg, 1000µg, 1100µg, 1200µg, 1400µg or 1600µg.

The present invention also relates to the use of (i) a polymer, or (ii) a silicon-based inorganic adsorbent for stabilizing amorphous selexipag.

### Process for the preparation of the crystalline solvates of Selexipag

Crystalline solvate of selexipag with aromatic hydrocarbon can be provided in step (a) for the preparation of amorphous solid dispersion of selexipag.

The present invention also relates to a process for the preparation of crystalline solvates of selexipag with aromatic hydrocarbon, the process comprising the steps of:
(i) providing a solution of selexipag in the organic hydrocarbon,
(ii) optionally adding seed crystals,
(iii) submitting the solution to crystallization conditions,
(iv) optionally isolating the crystalline solvate in solid form, and
(v) optionally drying the crystalline solvate.

The organic solvent in step (i) is preferably selected from aromatic hydrocarbons, preferably from chlorobenzene, toluene and xylene, more preferably from chlorobenzene, toluene and oxylene. Optionally, seed crystals may be added in step (ii). The seed crystals are prepared with the same solvent as the one used in step (i) of the process. In particular, the seed crystals are prepared using the same process steps as the solvates of the present invention. The seed crystals are typically added in an amount of 0.1 wt% to 10 wt%, preferably in an amount of 0.5 wt% to 7.0 wt%, most preferably 1.0 wt.% 5.0 wt%, on the basis of the total amount of the starting material used in step (i).

In step (iii) the solution is cooled to a temperature in the range of from -5 to -20 °C and kept at this temperature, typically for a period of time of from 2 to 24 hours, under stirring condition or without stirring, in order to promote crystallization.

Optionally, isolation in step (iv) may be performed by using procedures known in the art, such as by filtration, centrifugation, or evaporation of solvent. Moreover, the isolated crystals may optionally be dried in step (v), e.g. under reduced pressure, typically at room temperature, or heated up to a temperature between 25°C and 40°C or the crystals may directly be used in further processes, such as the preparation of amorphous solid dispersion of selexipag or isolated crystals may be used as seed crystals for the preparation of a solvate of selexipag.

### Crystalline solvates of Selexipag

The present invention also refers to new crystalline forms of selexipag. In particular, the present invention refers to new crystalline solvates of selexipag. These new solvate forms are preferably prepared from aromatic hydrocarbons, preferably from chlorobenzene, toluene and xylene, more preferably from chlorobenzene, toluene and o-xylene.

In another preferred embodiment, the solvate of selexipag of the present invention may be characterized by a stoichiometric composition of the solvates, i.e. number of solvent molecules per selexipag molecule as follows:
o-Xylene solvate (prepared according to Example 2 herein): 1.0 mol (±0.2 mol) of o-xylene,
Chlorobenzene solvate (prepared at -10°C under stirring, according to Example 3-1 herein): 1.0 mol (±0.2 mol) of chlorobenzene,
Chlorobenzene solvate (prepared at -18°C without stirring, according to Example 3-2 herein): 1.0 mol (±0.2 mol) of chlorobenzene,
Toluene solvate (prepared according to Example 4 herein): 1.0 mol (±0.2 mol) of toluene.

Thus, in one aspect described herein, the present invention refers to an o-xylene solvate of selexipag, having a characteristic powder X-ray diffractogram (PXRD) pattern with reflections at 2-theta values of 5.7±0.2, 13.3±0.2, 17.7±0.2, 19.2±0.2 and 20.7±0.2° 2-Theta, when measured at a temperature in the range of from 15 to 25°C with Cu-Kalpha1,2 radiation having a wavelength of 0.15419 nm. Thermogravimetric analysis (TGA) of o-xylene solvate of selexipag indicates a total weight loss between 45 °C and 220 °C of ca. 17.0%. (1.0 mole equivalent of o-xylene corresponds to ca. 17.6%).

In one aspect described herein, the present invention refers to a chlorobenzene solvate of selexipag, prepared under stirring conditions at a temperature of -10°C, having a characteristic powder X-ray diffractogram (PXRD) pattern with reflections at 2-theta values of 6.2±0.2, 10.5±0.2, 13.9±0.2, 18.8±0.2 and 19.3±0.2° 2-Theta, when measured at a temperature in the range of from 15 to 25°C with Cu-Kalpha1,2 radiation having a wavelength of 0.15419 nm. TGA of chlorobenzene solvate of selexipag, prepared under stirring conditions at a temperature of -10°C, indicates a total weight loss between 45 °C and 240 °C of ca. 18.0%. (1.0 mole equivalent of chlorobenzene corresponds to ca. 18.5%).

In one aspect described herein, the present invention refers to a chlorobenzene solvate of selexipag, prepared at a temperature of -18°C without stirring, having a characteristic powder X-ray diffractogram (PXRD) pattern with reflections at 2-theta values of 6.7±0.2, 17.0±0.2, 17.9±0.2, 19.4±0.2 and 24.5±0.2° 2-Theta, when measured at a temperature in the range of from 15 to 25°C with Cu-Kalpha1,2 radiation having a wavelength of 0.15419 nm. TGA of chlorobenzene solvate of selexipag, prepared at a temperature of -18°C without stirring, indicates a total weight loss between 50 °C and 220 °C of ca. 17.8%. (1.0 mole equivalent of chlorobenzene corresponds to ca. 18.5%).

In one aspect described herein, the present invention refers to a toluene solvate of selexipag, having a characteristic powder X-ray diffractogram (PXRD) pattern with reflections at 2-theta values of 5.8±0.2, 13.4±0.2, 17.9±0.2, 19.6±0.2, and 20.9±0.2° 2-Theta, when measured at a temperature in the range of from 15 to 25°C with Cu-Kalpha1,2 radiation having a wavelength of 0.15419 nm. TGA of toluene solvate of selexipag indicates a total weight loss between 40 °C and 240 °C of ca. 15.0%. (1.0 mole equivalent of toluene corresponds to ca. 15.6%).

### EXAMPLES

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions. A typical precision of the 2-Theta values is in the range of ± 0.2° 2-Theta. Thus, a diffraction peak that appears for example at 9.0 ° 2-theta (for crystalline form II of selexipag) can appear between 8.8 and 9.2 ° 2-theta on most X-ray diffractometers under standard conditions.

DSC was performed on a Mettler Polymer DSC R instrument. The sample was heated in a 40 microL aluminum pan with pierced aluminum lid from 25 to 250 °C at a rate of 10 K/min. Nitrogen (purge rate 50 mL/min) was used as purge gas.

TGA was performed on a Mettler TGA/DSC 1 instrument. The sample was heated in a 100 microL aluminum pan closed with an aluminum lid. The lid was automatically pierced at the beginning of the measurement. The sample was heated from 25 to 250 °C at a rate of 10 K/min. Nitrogen (purge rate 50 mL/min) was used as purge gas.

Moisture stability at accelerated stress conditions was performed in a Memmert constant climate chamber HPP110. 30-50 mg of a given solid composition prepared according to the Examples and Comparative Example herein were exposed to an atmosphere having a relative humidity of 75±0.5 % and a temperature of 40±0.5 °C for a period of time as indicated in Tables 5-7 below and analyzed via PXRD as described above.

### Example 1: Preparation of an amorphous solid dispersion comprising selexipag and HPMCAS

Selexipag (200 mg, e.g. prepared according to the procedure disclosed in example 84 WO 2002/088084 A1) or selexipag solvate (ca. 200-250 mg, e.g. prepared according to Examples 2 to 4 herein) and HPMCAS (200 mg; i.e. weight ration HPMCAS/Selexipag: 1.0 ±0.2) were dissolved in dichloromethane (5 mL). The solvent was removed on a rotary evaporator (bath temperature: 40 °C, final vacuum: 20 mbar) and the remaining solid was dried under vacuum (30 mbar) for two hours at 40 °C followed by 15 to 18 hours of drying under vacuum (30 mbar) at room temperature, yielding an amorphous solid dispersion of selexipag.

### Example 1-1

Selexipag (202 mg, prepared according to the procedure disclosed in example 84 WO 2002/088084 A1) and HPMCAS (206 mg) were dissolved in dichloromethane (5 mL). The solvent was removed on a rotary evaporator (bath temperature: 40 °C, final vacuum: 20 mbar) and the remaining solid was dried under vacuum (30 mbar) for two hours at 40 °C followed by 18 hours of drying under vacuum (30 mbar) at room temperature, yielding an amorphous solid dispersion of selexipag. The resulting solid dispersion was subjected to a moisture stability test as described in Example part herein. The results are summarized in Table 6 below. Representative PXRD pattern of the solid dispersion after its preparation is shown in Figure 3, the PXRD pattern of the solid dispersion after 6 weeks upon storage at a temperature of 40 °C and a relative humidity of 75% is shown in Figure 4. Figures 3 and 4 show that the selexipag comprised in the solid dispersion was in amorphous form and did not crystallize during the moisture stability test.

### Example 1-2

Chlorobenze solvate of selexipag (224 mg, prepared according to Example 3 herein) and HPMCAS (211 mg) were dissolved in dichloromethane (5 mL). The solvent was removed on a rotary evaporator (bath temperature: 40 °C, final vacuum: 20 mbar) and the remaining solid was dried under vacuum (30 mbar) for two hours at 40 °C followed by 18 hours of drying under vacuum (30 mbar) at room temperature, yielding an amorphous solid dispersion of selexipag. The resulting solid dispersion was subjected to a moisture stability test as described in Example part herein. The PXRD pattern of the solid dispersion after its preparation was essentially identical to the one shown in Figure 3, the PXRD pattern of the solid dispersion after 6 weeks upon storage at a temperature of 40 °C and a relative humidity of 75% was essentially identical to the one shown in Figure 4. Selexipag comprised in the solid dispersion was in amorphous form and did not crystallize during the moisture stability test.

### Example 1-3

o-Xylene solvate of selexipag (202 mg, prepared according to prepared according to Example 2 herein) and HPMCAS (207 mg) were dissolved in dichloromethane (5 mL). The solvent was removed on a rotary evaporator (bath temperature: 40 °C, final vacuum: 20 mbar) and the remaining solid was dried under vacuum (30 mbar) for two hours at 40 °C followed by 18 hours of drying under vacuum (30 mbar) at room temperature, yielding an amorphous solid dispersion of selexipag.

### Example 1-4

Toluene solvate of selexipag (240 mg, prepared according to prepared according to Example 4 herein) and HPMCAS (200 mg) were dissolved in dichloromethane (5 mL). The solvent was removed on a rotary evaporator (bath temperature: 40 °C, final vacuum: 20 mbar) and the remaining solid was dried under vacuum (30 mbar) for two hours at 40 °C followed by 17 hours of drying under vacuum (30 mbar) at room temperature, yielding an amorphous solid dispersion of selexipag.

### Example 2: Preparation of crystalline selexipag o-xylene solvate

Amorphous selexipag (314 mg, for example prepared according to the procedure described in comparative example 1 herein) was dissolved in 2.0 mL o-xylene at room temperature. The obtained clear solution was cooled to a temperature of about -9° C and stirred at this temperature for 20 hours in order to promote crystallization. To allow better stirring of the slurry, additional 2.0 mL o-xylene were added after crystallization took place. The obtained solid was collected by filtration and dried at room temperature under vacuum (about 30 mbar) for 18 hours to yield 301 mg selexipag o-xylene solvate.

PXRD: as depicted in Figure 5. The corresponding peak list is provided in Table 1 below.

**Table 1: Peak list of the PXRD pattern of the crystalline selexipag o-xylene solvate prepared according to Example 2 (relative peak intensities can vary due to e.g. inter-apparatus variability, sample crystallinity, sample preparation, etc.)**

| **position / ° 2 -theta** | **relative intensity / %** | | **position / ° 2 -theta** | **relative intensity / %** |
|---|---|---|---|---|
| 5,7±0.2 | 50 | | 22,5 ± 0.2 | 11 |
| 7,3 ± 0.2 | 11 | | 23,0±0.2 | 21 |
| 11,2 ± 0.2 | 10 | | 23,5 ± 0.2 | 9 |
| 11,7 ± 0.2 | 4 | | 24,4 ± 0.2 | 33 |
| 12,9 ± 0.2 | 71 | | 24,7 ± 0.2 | 20 |
| 13,3 ± 0.2 | 100 | | 25,0 ± 0.2 | 22 |
| 13,8 ± 0.2 | 4 | | 25,3 ± 0.2 | 53 |
| 15,2 ± 0.2 | 32 | | 25,9 ± 0.2 | 43 |
| 15,6 ± 0.2 | 43 | | 27,1 ± 0.2 | 12 |
| 16,1 ± 0.2 | 6 | | 28,0 ± 0.2 | 8 |
| 17,1 ± 0.2 | 94 | | 28,4 ± 0.2 | 7 |
| 17,7 ± 0.2 | 91 | | 29,1 ± 0.2 | 12 |
| 18,0 ± 0.2 | 7 | | 29,8 ± 0.2 | 5 |
| 18,4 ± 0.2 | 55 | | 30,7 ± 0.2 | 4 |
| 19,2 ± 0.2 | 53 | | 31,4 ± 0.2 | 9 |
| 19,5 ± 0.2 | 24 | | 32,1 ± 0.2 | 4 |
| 19,8 ± 0.2 | 57 | | 32,5 ± 0.2 | 3 |
| 20,1 ± 0.2 | 13 | | 33,2 ± 0.2 | 3 |
| 20,7 ± 0.2 | 32 | | 34,7 ± 0.2 | 8 |
| 21,5 ± 0.2 | 5 | | 35,7 ± 0.2 | 5 |

DSC: DSC curve shows a first endotherm with an onset temperature of about 65 °C (peak maximum at about 69 °C), corresponding to sample desolvation. Further heating leads to sample conversion to crystalline form I of selexipag. Melting endotherm of form I is then observed with an onset temperature of about 134 °C (peak maximum at about 137 °C).

TGA: TGA curve indicates a total weight loss between 45 °C and 220 °C of ca. 17.0%. (1.0 mole equivalent of o-xylene corresponds to ca. 17.6%).

### Example 3: Preparation of crystalline selexipag chlorobenzene solvates

### Example 3-1

Amorphous selexipag (302 mg, for example prepared according to the procedure described in comparative example 1 herein) was dissolved in 1.5 mL chlorobenzene at room temperature. The obtained clear solution was cooled to a temperature of about -10 °C and stirred at this temperature for 20 hours in order to promote crystallization. The obtained solid was collected by filtration and dried at room temperature under vacuum (about 30 mbar) for 18 hours to yield 262 mg selexipag chlorobenzene solvate.

PXRD: as depicted in Figure 6. The corresponding peak list is provided in Table 2 below.

**Table 2: Peak list of the PXRD pattern of the crystalline selexipag chlorobenzene solvate prepared according to Example 3-1 (relative peak intensities can vary due to e.g. inter-apparatus variability, sample crystallinity, sample preparation, etc.)**

| **position / ° 2 -theta** | **relative intensity / %** | | **position / ° 2 -theta** | **relative intensity / %** |
|---|---|---|---|---|
| 6,2±0.2 | 88 | | 22,3 ± 0.2 | 16 |
| 6,8±0.2 | 27 | | 22,6 ± 0.2 | 10 |
| 10,5 ± 0.2 | 8 | | 22,9 ± 0.2 | 6 |
| 11,7 ± 0.2 | 14 | | 23,2 ± 0.2 | 23 |
| 12,2 ± 0.2 | 10 | | 23,4 ± 0.2 | 16 |
| 12,6 ± 0.2 | 3 | | 23,9 ± 0.2 | 16 |
| 13,1 ± 0.2 | 10 | | 24,2 ± 0.2 | 5 |
| 13,6 ± 0.2 | 10 | | 24,6 ± 0.2 | 48 |
| 13,9 ± 0.2 | 19 | | 25,1 ± 0.2 | 11 |
| 14,8 ± 0.2 | 5 | | 26,0 ± 0.2 | 14 |
| 15,0 ± 0.2 | 17 | | 26,1 ± 0.2 | 21 |
| 15,3 ± 0.2 | 14 | | 27,3 ± 0.2 | 2 |
| 15,5 ± 0.2 | 15 | | 28,1 ± 0.2 | 3 |
| 16,1 ± 0.2 | 23 | | 28,5 ± 0.2 | 4 |
| 16,2 ± 0.2 | 9 | | 29,7 ± 0.2 | 4 |
| 16,7 ± 0.2 | 3 | | 30,3 ± 0.2 | 4 |
| 18,3 ± 0.2 | 27 | | 30,9 ± 0.2 | 6 |
| 18,8 ± 0.2 | 75 | | 31,3 ± 0.2 | 6 |
| 19,3 ± 0.2 | 100 | | 33,1 ± 0.2 | 5 |
| 20,2 ± 0.2 | 8 | | 34,6 ± 0.2 | 5 |
| 21,2 ± 0.2 | 9 | | | |

DSC: DSC curve shows a first endotherm with an onset temperature of about 66 °C (peak maximum at about 69 °C), corresponding to sample desolvation. Further heating leads to sample conversion to crystalline form I of selexipag. Melting endotherm of form I is then observed with an onset temperature of about 134 °C (peak maximum at about 139 °C).

TGA: TGA curve of chlorobenzene solvate of selexipag, prepared under stirring conditions at a temperature of -10°C, indicates a total weight loss between 45 °C and 240 °C of ca. 18.0%. (1.0 mole equivalent of chlorobenzene corresponds to ca. 18.5%).

### Example 3-2

Amorphous selexipag (308 mg, for example prepared according to the procedure described in comparative example 1 herein) was dissolved in 1.5 mL chlorobenzene at room temperature. The obtained clear solution was cooled to a temperature of about -18 °C and let stand at this temperature without stirring for 24 hours in order to promote crystallization. The obtained solid was collected by filtration and dried at room temperature under vacuum (about 30 mbar) for 18 hours to yield 266 mg selexipag chlorobenzene solvate.

PXRD: as depicted in Figure 7. The corresponding peak list is provided in Table 3 below.

**Table 3: Peak list of the PXRD pattern of the crystalline selexipag chlorobenzene solvate prepared according to Example 3-2 (relative peak intensities can vary due to e.g. inter-apparatus variability, sample crystallinity, sample preparation, etc.)**

| **position / ° 2 -theta** | **relative intensity / %** | | **position / ° 2 -theta** | **relative intensity / %** |
|---|---|---|---|---|
| 6,2 ± 0.2 | 45 | | 22,8 ± 0.2 | 13 |
| 6,7 ± 0.2 | 46 | | 23,0 ± 0.2 | 30 |
| 10,3 ± 0.2 | 4 | | 23,5 ± 0.2 | 35 |
| 11,4 ± 0.2 | 7 | | 23,8 ± 0.2 | 12 |
| 11,7 ± 0.2 | 9 | | 24,5 ± 0.2 | 76 |
| 12,2 ± 0.2 | 6 | | 24,9 ± 0.2 | 12 |
| 12,8 ± 0.2 | 34 | | 25,5 ± 0.2 | 29 |
| 13,1 ± 0.2 | 21 | | 26,0 ± 0.2 | 44 |
| 13,4 ± 0.2 | 15 | | 26,9 ± 0.2 | 19 |
| 13,7 ± 0.2 | 43 | | 27,1 ± 0.2 | 15 |
| 14,4 ± 0.2 | 11 | | 27,7 ± 0.2 | 5 |
| 14,7 ± 0.2 | 43 | | 28,0 ± 0.2 | 6 |
| 15,4 ± 0.2 | 4 | | 28,3 ± 0.2 | 6 |
| 15,8 ± 0.2 | 9 | | 28,7 ± 0.2 | 22 |
| 16,1 ± 0.2 | 17 | | 29,7 ± 0.2 | 11 |
| 17,0 ± 0.2 | 57 | | 31,0 ± 0.2 | 7 |
| 17,9 ± 0.2 | 100 | | 31,9 ± 0.2 | 6 |
| 18,4 ± 0.2 | 15 | | 32,6 ± 0.2 | 8 |
| 19,1 ± 0.2 | 52 | | 34,2 ± 0.2 | 5 |
| 19,4 ± 0.2 | 72 | | 35,0 ± 0.2 | 5 |
| 19,7 ± 0.2 | 55 | | 35,5 ± 0.2 | 4 |
| 20,2 ± 0.2 | 6 | | 37,2 ± 0.2 | 5 |
| 20,7 ± 0.2 | 16 | | 38,5 ± 0.2 | 3 |
| 21,1 ± 0.2 | 8 | | 39,1 ± 0.2 | 4 |
| 22,2 ± 0.2 | 5 | | 39,1 ± 0.2 | 9 |
| 22,4 ± 0.2 | 13 | | | |

DSC: DSC curve shows a first endotherm with an onset temperature of about 61 °C (peak maximum at about 64 °C), corresponding to sample desolvation. Further heating leads to sample conversion to crystalline form I of selexipag. Melting endotherm of form I is then observed with an onset temperature of about 137 °C (peak maximum at about 139 °C).

TGA: TGA curve of chlorobenzene solvate of selexipag, prepared at a temperature of -18°C without stirring, indicates a total weight loss between 50 °C and 220 °C of ca. 17.8%. (1.0 mole equivalent of chlorobenzene corresponds to ca. 18.5%).

### Example 4: Preparation of crystalline selexipag toluene solvate

Amorphous selexipag (305 mg, for example prepared according to the procedure described in comparative example 1 herein) was dissolved in 2.0 mL toluene at room temperature. The obtained clear solution was cooled to a temperature of about -10 °C and stirred at this temperature for 20 hours in order to promote crystallization. To allow better stirring of the slurry, additionnal 1.0 mL toluene was added after crystallization took place. The obtained solid was collected by filtration and dried at room temperature under vacuum (about 30 mbar) for 18 hours to yield 322 mg selexipag toluene solvate.

PXRD: as depicted in Figure 8. The corresponding peak list is provided in Table 4 below.

**Table 4: Peak list of the PXRD pattern of the crystalline selexipag toluene solvate prepared according to Example 4 (relative peak intensities can vary due to e.g. inter-apparatus variability, sample crystallinity, sample preparation, etc.)**

| **position / ° 2 -theta** | **relative intensity / %** | | **position / ° 2 -theta** | **relative intensity / %** |
|---|---|---|---|---|
| 5,8 ± 0.2 | 100 | | 22,5 ± 0.2 | 2 |
| 7,2 ± 0.2 | 13 | | 23,5 ± 0.2 | 10 |
| 10,6 ± 0.2 | 2 | | 23,8 ± 0.2 | 9 |
| 11,1 ± 0.2 | 11 | | 24,1 ± 0.2 | 2 |
| 12,9 ± 0.2 | 23 | | 24,5 ± 0.2 | 4 |
| 13,4 ± 0.2 | 44 | | 24,7 ± 0.2 | 8 |
| 13,9 ± 0.2 | 4 | | 25,2 ± 0.2 | 11 |
| 15,6 ± 0.2 | 31 | | 25,3 ± 0.2 | 14 |
| 16,3 ± 0.2 | 11 | | 25,7 ± 0.2 | 14 |
| 17,1 ± 0.2 | 30 | | 25,9 ± 0.2 | 6 |
| 17,9 ± 0.2 | 44 | | 26,1 ± 0.2 | 15 |
| 18,3 ± 0.2 | 43 | | 26,8 ± 0.2 | 8 |
| 19,6 ± 0.2 | 43 | | 27,8 ± 0.2 | 4 |
| 19,8 ± 0.2 | 41 | | 28,7 ± 0.2 | 2 |
| 20,5 ± 0.2 | 6 | | 29,1 ± 0.2 | 5 |
| 20,9 ± 0.2 | 21 | | 29,6 ± 0.2 | 3 |
| 21,3 ± 0.2 | 8 | | 30,1 ± 0.2 | 1 |
| 21,9 ± 0.2 | 2 | | 31,3 ± 0.2 | 8 |
| 22,3 ± 0.2 | 5 | | 32,6 ± 0.2 | 2 |

DSC: DSC curve shows a first endotherm with an onset temperature of about 69 °C (peak maximum at about 72 °C), corresponding to sample desolvation. Further heating leads to sample conversion to crystalline form I of selexipag. Melting endotherm of form I is then observed with an onset temperature of about 137 °C (peak maximum at about 141 °C).

TGA: TGA curve indicates a total weight loss between 40 °C and 240 °C of ca. 15.0%. (1.0 mole equivalent of toluene corresponds to ca. 15.6%).

### Comparative example 1: Physical stability of amorphous selexipag at accelerated stress conditions

Selexipag (1g, e.g. prepared according to the procedure disclosed in example 84 WO 2002/088084 A1) was dissolved in dichloromethane (10 mL). The solvent was removed on a rotary evaporator (40 °C bath temperature, 20 mbar final vacuum) and the remaining solid was dried at room temperature under vacuum (about 30 mbar) for about 18 hours. PXRD depicted in Figure 1 confirmed the receipt of amorphous selexipag. The obtained amorphous selexipag was open stored at accelerated stress conditions (temperature: 40 °C, relative humidity: 75%). The material was analyzed by powder X-ray diffraction after one, three, and six weeks and the results are summarized in Table 5.

**Table 5: Results of stress test with neat amorphous selexipag (am = amorphous, II = crystalline form II of WO 2010/150865 A1, III = crystalline form III of WO 2010/150865 A1)**

| **PXRD analysis after** | | | |
|---|---|---|---|
| start | 1 week | 3 weeks | 6 weeks |
| am | am + III + II | II+III+am | II+III+am |

As can be seen from Table 5 neat amorphous selexipag is physically unstable and readily crystallizes during the stress test, whereat crystallinity increases gradually.

PXRD pattern of the selexipag sample after 6 weeks upon storage at a temperature of 40 °C and a relative humidity of 75% is shown in Figure 2.

### Example 5: Physical stability of solid dispersions with amorphous selexipag and various polymers at accelerated stress conditions

Selexipag (200 mg e.g. prepared according to the procedure disclosed in example 84 WO 2002/088084 A1) and the polymer (200 mg) according to Table 6 were dissolved in dichloromethane (5 mL in Examples A and B, 6 mL in Example C). The solvent was removed on a rotary evaporator (bath temperature: 40 °C, final vacuum: 20 mbar) and the remaining solid was dried under vacuum (30 mbar) for two hours at 40 °C followed by 15 to 18 hours of drying at room temperature. The so obtained solid dispersions were open stored at accelerated stress conditions (temperature: 40 °C, relative humidity: 75%) for six weeks. The samples were periodically analyzed by powder X-ray diffraction and the results are summarized in Table 6.

**Table 6: Results of stress tests with solid dispersions of amorphous selexipag and polymer carrier (am = amorphous, II = crystalline form II of WO 2010/150865 A1))**

| **Example** | **Polymer carrier** | **PXRD analyses after** | | | |
|---|---|---|---|---|---|
| | | 1 week | 2 weeks | 3 weeks | 6 weeks |
| A | PVP (40000) | am | am +traces crystalline | am + traces II | am + II |
| B | Soluplus | am | am | am | am |
| C | HPMCAS | am | am | am | am |

In contrast to solid dispersions prepared with Soluplus and hydroxypropylmethylcellulose acetate (HPMCA), stabilization with polyvinylpyrrolidon (PVP 40000) was not as successful since traces of a crystalline phase were already visible in the two weeks sample.

### Example 6: Physical stability of solid dispersions with amorphous selexipag and various silicon-based carriers at accelerated stress conditions

Selexipag (200 mg e.g. prepared according to the procedure disclosed in example 84 WO 2002/088084 A1) and silicon-based carrier (200 mg) according to Table 7 were suspended in dichloromethane (5 mL). The solvent was removed on a rotary evaporator (bath temperature: 40 °C, final vacuum: 20 mbar) and the remaining solid was dried under vacuum for one to two hours at 40 °C followed by 15 to 18 hours of drying at room temperature. The so obtained solid dispersions were open stored at accelerated stress conditions (temperature: 40 °C, relative humidity: 75%). The samples were periodically analyzed by powder X-ray diffraction and the results are summarized in Table 7.

**Table 7: Results of stress tests with solid dispersions of amorphous selexipag and silica carrier (am = amorphous)**

| **Example** | **Silicon-based carrier** | **PXRD analyses after** | | | |
|---|---|---|---|---|---|
| | | 1 week | 2 weeks | 3 weeks | 6 weeks |
| A | Neusilin UFL2 | am | am | am + traces | am + traces |
| B | Syloid 72 FP | am | am | crystalline am | crystalline am |

## Claims

1. A solid dispersion comprising amorphous 2-[4-[*N*-(5,6-diphenylpyrazin-2-yl)-*N-*isopropylamino]butyloxy]-*N*-(methylsulfonyl)acetamide (selexipag) and at least one pharmaceutically acceptable excipient, wherein the at least one pharmaceutically acceptable excipient is (i) a polymer, or (ii) a silicon-based inorganic adsorbent.

2. The solid dispersion according to claim 1, not comprising crystalline selexipag.

3. The solid dispersion according to any one of claims 1 to 2, wherein the solid dispersion is amorphous.

4. The solid dispersion according to any one of claims 1 to 3, wherein the pharmaceutically acceptable excipient is an organic polymer.

5. The solid dispersion according to claim 4, wherein the organic polymer is selected from the group consisting of chemically modified cellulose, cellulose ether, polyvinylpyrrolidone, polyethylene glycol, polyethylene glycol based copolymer, polyacrylic acids and salts thereof, polyvinylalcohol, polyacrylamides copolymer, methacrylic acid copolymer, methacrylate copolymer, pectines, chitin and chitosan derivatives, polyphosphates, polyoxazoline and polysaccharides.

6. The solid dispersion according to claim 5, wherein the chemically modified cellulose is hydroxypropylmethylcellulose acetate succinate (HPMCAS).

7. The solid dispersion according to claim 5, wherein the organic polymer is polyethylene glycol based copolymer is a polyethyleneglycol- polyvinyl caprolactam-polyvinyl acetate grafted copolymer.

8. The solid dispersion according to any one of claims 1 to 3, wherein the pharmaceutically acceptable excipient is a silicon-based inorganic compound selected from silica and aluminosilicates.

9. The solid dispersion according to any one of the preceding claims, wherein the weight ratio of selexipag and the pharmaceutically acceptable excipient is from 1.0: 0.5 to 1.0 : 8.0, and wherein the weight ratio is calculated based on the total amount of selexipag present in said solid dispersion and on the total amount of the pharmaceutically acceptable excipients present in said solid dispersion.

10. A pharmaceutical composition comprising a solid dispersion according to any one of claims 1 to 9, and further comprising one or more additional pharmaceutically acceptable excipients.

11. A process for the preparation of the solid dispersion according to any one of claims 1 to 10 comprising:
a) providing selexipag;
b) dissolving or dispersing selexipag provided in step (a) and the at least one pharmaceutically acceptable excipient, wherein the at least one pharmaceutically acceptable excipient is (i) a polymer, or (ii) a silicon-based inorganic adsorbent,
in a solvent to form a mixture, and
c) removing at least part, preferably essentially all, of the solvent to provide the solid dispersion.

12. The process according to claim 11, wherein the at least one pharmaceutically acceptable excipient is an organic polymer and wherein step b) results in a solution.

13. The process according to claim 11, wherein the at least one pharmaceutically acceptable excipient is a silicon-based inorganic compound and wherein step b) results in a suspension.

14. The process according to any one of claims 11 to 13, wherein the solvent in step (b) is selected from an alcohol, a ketone, an ester, a halogenated hydrocarbon, a nitrile and an ether, preferably wherein the solvent in step (b) is a halogenated hydrocarbon or a ketone.

15. Use of the solid dispersion as defined in any one of claims 1 to 9 for the preparation of a pharmaceutical composition.
